# EUROPÄISCHE PATENTSCHRIFT

(11) **EP 1 622 877 B1**
(45) Veröffentlichungstag und Bekanntmachung des Hinweises auf die Patenterteilung: **20.09.2006**
(21) Anmeldenummer: 04741484.2
(22) Anmeldetag: 27.04.2004
(51) Int. Cl.: C07D 233/54

(54) **VERFAHREN ZUR HERSTELLUNG VON IONISCHEN FLÜSSIGKEITEN MIT ALKYLSULFAT UND FUNKTIONALISIERTEN ALKYLSULFAT-ANIONEN**
METHOD FOR THE PRODUCTION OF IONIC LIQUIDS CONTAINING ALKYL SULPHATE AND FUNCTIONALISED ALKYL SULPHATE-ANIONS
PROCEDE DE PRODUCTION DE LIQUIDES IONIQUES AVEC DU SULFATE D'ALKYLE ET DES ANIONS DE SULFATE D'ALKYLE FONCTIONNALISES

(30) Priorität: 29.04.2003 DE 10319465
(43) Veröffentlichungstag der Anmeldung: 08.02.2006
(73) Patentinhaber: Solvent Innovation GmbH, 50829 Köln (DE)
(72) Erfinder: WASSERSCHEID, Peter, 50829 Koeln (DE); VAN HAL, Roy, NL-6451 HD Schinveld (NL); HILGERS, Claus, 50829 Koeln (DE)
(74) Vertreter: Weber, Thomas
(86) Internationale Anmeldenummer: PCT/EP2004/050619
(87) Internationale Veröffentlichungsnummer: WO 2004/096776

(56) Entgegenhaltungen:
- WASSERSCHEID P ET AL: "1-N-BUTYL-3-METHYLIMIDAZOLIUM (ÄBMIMÜ) OCTYLSULFATE-AN EVEN 'GREENER' IONIC LIQUID" GREEN CHEMISTRY, ROYAL SOCIETY OF CHEMISTRY, CAMBRIDGE,, GB, Bd. 4, Nr. 4, 2002, Seiten 400-404, XP008024341 ISSN: 1463-9262
- DATABASE BEILSTEIN BEILSTEIN INSTITUTE FOR ORGANIC CHEMISTRY, FRANKFURT-MAIN, DE; 19. März 1991 (1991-03-19), XP002295666 Database accession no. PRODUCT BRN 4083386 & OLIVEROS ET AL: BULL. SOC. CHIM. FR., 1969, Seiten 2815-2820,

## Beschreibung

Die Erfindung betrifft ein Verfahren zur Herstellung von ionischen Flüssigkeiten der allgemeinen Formel [Kation][R'-O-SO₃]. Das erfindungsgemäße Verfahren erlaubt die Herstellung dieser ionischen Flüssigkeiten in bisher unerreichter Qualität ausgehend von technisch verfügbaren Rohstoffen und kann leicht in den technischen Produktionsmaßstab überführt werden.

Ionische Flüssigkeiten mit Alkylsulfat- und funktionalisierten Alkylsulfaten sind als halogenfreie Lösungsmittel, Extraktionsmittel und Wärmeträger von erheblicher technischer Bedeutung. Während ionische Flüssigkeiten mit Methyl- und Ethylsulfat-Anionen bekannterweise leicht durch Alkylierung eines Imidazols, Pyridins, Amins oder Phosphans mit Dimethylsulfat oder Diethylsulfat zugänglich sind (P. Wasserscheid, C. Hilgers, EP-A-1182196), ist die Herstellung aller übrigen Vertreter nach bekanntem Verfahren bisher sehr aufwendig und führt häufig zu mehr oder weniger verunreinigten Produkten.

Bei der Herstellung von 1-Butyl-3-methylimidazolium Octylsulfat wird nach dem Stand der Technik das Chlorid-Salz des 1-Butyl-3-methylimidazolium-Kations mit Natriumoctylsulfat unter Fällung oder Extraktion von Natriumchlorid umgesetzt (P. Wasserscheid, R. van Hal, A. Bösmann, Green Chem. 2002, 4(4), 400-404). Diese Metathese-Reaktion ist im Grunde auch zur Herstellung anderer ionischer Flüssigkeiten mit Alkylsulfat- und funktionalisierten Alkylsulfat-Ionen geeignet, allerdings erfordert die Abtrennung des Alkalimetallhalogenids aufwendige Extraktions- oder Filtrationsschritte. Ferner lässt sich selbst beim Arbeiten mit absolutierten Lösungsmitteln nicht vermeiden, dass Verunreinigungen von Halogenid- und/oder Alkalimetallionen ins Produkt gelangen.

Ausgehend vom Stand der Technik war es eine Aufgabe der vorliegenden Erfindung ein Verfahren zur Herstellung von ionischen Flüssigkeiten der allgemeinen Formel [Kation][R'-O-SO₃] zur Verfügung zu stellen, mit welchem die ionischen Flüssigkeiten ausgehend von leicht verfügbaren Rohstoffen in hoher Qualität, d.h. in großer Reinheit, auch ohne Weiteres im großen Maßstab hergestellt werden können.

Gemäß der Erfindung wurde dieses Problem gelöst durch ein Verfahren zur Herstellung ionischer Flüssigkeiten der allgemeinen Formel [Kation][R'-O-SO₃], in der R' eine Gruppe der allgemeinen Formel R⁴-[X(-CH₂-)ₙ]ₘ darstellt, in der n eine Zahl zwischen 0 und 12 repräsentiert, m unabhängig von n eine Zahl zwischen 1 und 400 darstellt, X ausgewählt ist aus der Gruppe bestehend aus Sauerstoff, Schwefel, Selen, einer Einfachbindung oder einer Gruppe der allgemeinen Formel -O-Si(CH₃)₂-O-, -O-Si(CH₂CH₃)₂-O-, -O-Si(OCH₃)₂-O-, -0-Si(O-CH₂CH₃)₂-O-, -P(Phenyl)-, -PR''- und R⁴ eine lineare oder verzweigte, gesättigte oder ungesättigte, aliphatische oder alicyclische, unfunktionalisierte oder mit einer oder mehreren Gruppen Y funktionalisierte Alkylgruppe mit 1 bis 36 Kohlenstoffatomen darstellt wobei Y eine -OH, -OR'', -COOH, -COOR'', -NH₂, -SO₄, -F, -Cl, -Br, -I oder -CN -Gruppe ist und R'' eine verzweigte oder lineare Kohlenwasserstoffkette mit 1 bis 12 Kohlenstoffatomen repräsentiert, welches dadurch gekennzeichnet ist, dass
ein Salz der allgemeinen Formel [Kation][CH₃-O-SO₃] oder [Kation][CH₃-CH₂-O-SO₃] mit wenigstes einem Alkohol der allgemeinen Formel R'-OH umgesetzt wird, wobei
das [Kation] ausgewählt ist aus der Gruppe bestehend aus
- quartemären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 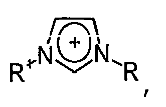 wobei der Imidazol-Kern substituiert sein kann mit wenigstens einer Gruppe, die ausgewählt ist aus C₁-C₆-Alkyl-, C₁-C₆-Alkoxy-, C₁-C₆₋Aminoalkyl-, C₅-C₁₂-Aryl- oder C₅-C₁₂-Aryl-C₁-C₆-Alkylgruppen,
- Pyridinium-Kationenen der allgemeinen Formel 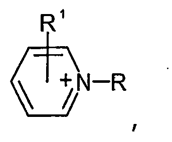 wobei der Pyridin-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆-Alkylgruppen,
- Pyrazolium-Kationenen der allgemeinen Formel 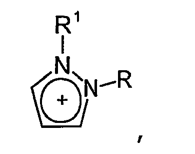 wobei der Pyrazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆-Alkylgruppen,
- und Triazolium-Kationenen der allgemeinen Formel 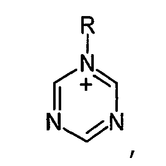 wobei der Triazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆-Alkylgruppen,
   und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
   und der Rest R ausgewählt ist aus
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenenatomen substituiert sein können,
   und R' die oben angegebene Bedeutung hat.

Bevorzugte Ausgestaltungen und Alternativen des erfindungsgemäßen Verfahrens ergeben sich aus den Patentansprüchen.

Die Umsetzung erfolgt vorzugsweise bei erhöhten Temperaturen unter Freisetzung von Methanol oder Ethanol, je nach dem welches Salz, [Kation][CH₃₋O-SO₃] oder [Kation][CH₃-CH₂-O-SO₃] eingesetzt wird. Der gebildete Ethanol bzw. das Methanol kann durch einfache Verfahren, wie Destillation etc, aus dem Reaktionsgemisch abgetrennt werden. Das erfindungsgemäße Verfahren ist insbesondere für Methylsulfat- und Ethylsulfat-Salze mit Pyridinium-, Imidazolium-, Ammonium- und Phosphonium-Ionen einsetzbar.

Das erfindungsgemäße Verfahren zeichnet sich gegenüber dem Stand der Technik dadurch aus, dass als Nebenprodukt der Umsetzung zur Bildung der ionischen Flüssigkeiten mit Alkylsulfat- und funktionalisierten Alkylsulfat-Ionen anstelle des nicht-flüchtigen und schwer-abtrennbaren Alkalimetallhalogenids die flüchtige Verbindung Methanol bzw. Ethanol gebildet wird, die sehr einfach und vollständig durch Destillation aus dem Produkt entfernt werden kann. Auf diese Weise erlaubt das erfindungsgemäße Verfahren eine Herstellung von ionischen Flüssigkeiten der allgemeinen Formel [Kation][R'-O-SO₃] in hochreiner Form, also in bisher unerreichter Qualität.

Ein weiterer Vorteil des erfindungsgemäßen Verfahrens besteht darin, dass das Verfahren auf Grund der großen Anzahl an technisch verfügbaren, funktionalisierten und nicht funktionalisierten Alkoholen R'-OH sehr universell eingesetzt werden kann, während man im Zusammenhang mit den alten Verfahren gemäß dem Stand der Technik auf die schlecht zugänglichen Alkalimetallsalze von Schwefelsäurehalbestem zurückgreifen muss.

Ein weiterer Vorteil des erfindungsgemäßen Verfahrens besteht darin, dass im Gegensatz zum Stand der Technik während der Herstellung auf aufwendige Extraktions- und Filtrationsschritte verzichtet werden kann. Dadurch lässt sich der Einsatz von Hilfsstoffen und Lösungsmitteln erheblich reduzieren, wodurch die Produktionskosten erheblich gesenkt und die Umweltbelastung minimiert werden kann. Ferner erlaubt das erfindungsgemäße Verfahren eine wesentlich einfachere Herstellung der ionischen Flüssigkeiten in größerem Maßstab.

In einer bevorzugten Ausführungsform ist R⁴ eine lineare oder verzweigte Alkylgruppe mit 1 bis 12, vorzugsweise von 1 bis 6, besonders bevorzugt von 1 bis 4 Kohlenstoffatomen. Beispielhaft seien die Methyl-, Ethyl-, Propyl-, iso-Propyl-, n-Butyl, i-Butyl-, oder tert.-Butyl-Gruppe genannt.

In einer bevorzugten Ausführungsform ist n eine ganze Zahl zwischen 2 und 10, besonders bevorzugt zwischen 3 und 7. Unabhängig davon ist in einer bevorzugten Ausführungsform m eine ganze Zahl zwischen 1 und 7, besonders bevorzugt zwischen 2 und 5.

In einer weiteren bevorzugten Ausführungsform ist X = Sauerstoff, n = 2 und m = 1 bis 6, vorzugsweise bis 5, und R⁴ ist eine lineare oder verzweigte Alkylgruppe mit 1 bis 12, vorzugsweise von 1 bis 6, besonders bevorzugt von 1 bis 4 Kohlenstoffatomen, beispielsweise die Methyl-, Ethyl-, Propyl-, iso-Propyl-, n-Butyl, i-Butyl-, oder tert.-Butyl-Gruppe.

In einer weiteren bevorzugten Ausführungsform ist X = eine Einfachbindung, n = 2 bis 12, vorzugsweise 3 bis 7 und m = 1 bis 6, vorzugsweise bis 5, und R⁴ ist eine lineare oder verzweigte Alkylgruppe mit 1 bis 12, vorzugsweise von 1 bis 6, besonders bevorzugt von 1 bis 4 Kohlenstoffatomen, beispielsweise die Methyl-, Ethyl-, Propyl-, iso-Propyl-, n-Butyl, i-Butyl-, oder tert.-Butyl-Gruppe.

In einer bevorzugten Ausführungsform ist R'' eine lineare oder verzweigte Alkylgruppe mit 1 bis 8, vorzugsweise von 1 bis 6, besonders bevorzugt von 1 bis 4 Kohlenstoffatomen. Beispielhaft seien die Methyl-, Ethyl-, Propyl-, iso-Propyl-, n-Butyl, i-Butyl-, oder tert.-Butyl-Gruppe genannt.

Es versteht sich von selbst, dass R' von einer Methyl- und Ethylgruppe verschieden ist.

Bevorzugte Kationen [Kation] gemäß der Erfindung sind ausgewählt aus der Gruppe bestehend aus [1,3-Dimethylimidazolium], [1,2,3-Trimethylimidazolium], [1-Ethyl-3-methylimidazolium], [1-Butyl-3-methylimidazolium], [1-Octyl-3-methylimidazolium], [1-Methylpyridinium] und [Trioctylmethylammonium]. Bevorzugte Anionen [R'-O-SO₃] gemäß der Erfindung sind ausgewählt aus der Gruppe bestehend aus [Me-(O-CH₂-CH₂)₂-O-SO₃], [Me-(O-CH₂-CH₂)₃-O-SO₃], [Me-(O-CH₂-CH₂)₄-O-SO₃], [Me-(O-CH₂-CH₂)₅-O-SO₃], [Et-(O-CH₂-CH₂)₂-O-SO₃], [Et-(O-CH₂-CH₂)₃-O-SO₃], [CH₃-(CH₂)₃-O-SO₃] und [CH₃-(CH₂)₇-O-SO₃].

In einer bevorzugten Ausführungsform des erfindungsgemäßen Verfahrens wird ein Salz [Kation][CH₃-OSO₃] oder ein Salz [Kation][CH₃CH₂-OSO₃] mit 1 bis 10 Äquivalenten, bevorzugt mit 1 bis 3 Äquivalenten, besonders bevorzugt mit 1 bis 2 Äquivalenten eines funktionalisierten oder nicht funktionalisierten Alkohols R'-OH bei einer Temperatur von 0 bis 250 °C, bevorzugt von 60 bis 180 °C, besonders bevorzugt von 80 bis 160 °C umgesetzt und anschließend oder zeitgleich zur Reaktion das gebildete Koppelprodukt Methanol bzw. Ethanol durch Destillation entfernt. In Fällen, in denen der Alkohol R'-OH im Überschuss eingesetzt wird, kann dieser Überschuss ebenfalls destillativ entfernt werden.

In einer besonderen Ausführungsform des erfindungsgemäßen Verfahrens wird während der Reaktion des Salzes [Kation][CH₃-OSO₃] bzw. des Salzes [Kation][CH₃CH₂-OSO₃] mit dem funktionalisierten oder nicht funktionalisierten Alkohol R'-OH ein Katalysator zugesetzt, um die Reaktion zu beschleunigen. Geeignete Katalysatoren sind feste oder flüssige, Lewis- oder Brønsted-Basen sowie feste oder flüssige, Lewis- oder Brønsted-Säuren. Besonders bevorzugte Katalysatoren sind Ionentauscherharze vom Amberlyst- oder Dowex-Typ, Zeolite sowie Übergangsmetallkomplexe des Titans, Zirkoniums, Eisens oder Kupfers sowie Phosphane.

In einer besonderen Ausführungsform des erfindungsgemäßen Verfahrens werden mehrere, vorzugsweise wenigstens zwei unterschiedliche Alkohole der Formel R'-OH gleichzeitig im erfindungsgemäßen Verfahren umgesetzt. Diese Verfahrensweise führt als Reaktionsprodukt zu einer ionischen Flüssigkeit, die ein Gemisch aus den unterschiedlichen funktionalisierten und nicht funktionalisierten Alkylsulfaten umfasst.

Explizit erwähnt seien folgende ionische Flüssigkeiten (Me- = Methylgruppe, Et- = Ethylgruppe), die unter Verwendung des erfindungsgemäßen Verfahrens hergestellt werden können.
[1,3-Dimethylimidazolium][Me-(O-CH₂-CH₂)₂-O-SO₃]
[1,3-Dimethylimidazolium][Me-(O-CH₂-CH₂)₃-O-SO₃]
[1,3-Dimethylimidazolium][Me-(O-CH₂-CH₂)₄-O-SO₃]
[1,3-Dimethylimidazolium][Me-(O-CH₂-CH₂)₅-O-SO₃]
[1,3-Dimethylimidazolium][Et-(O-CH₂-CH₂)₂-O-SO₃]
[1,3-Dimethylimidazolium][Et-(O-CH₂-CH₂)₃-O-SO₃]
[1,3-Dimethylimidazolium][CH₃-(CH₂)₃-O-SO₃]
[1,3-Dimethylimidazolium][CH₃-(CH₂)₇-O-SO₃]
[1,2,3-Trimethylimidazolium][Me-(O-CH₂-CH₂)₂-O-SO₃]
[1,2,3-Trimethylimidazolium][Me-(O-CH₂-CH₂)₃-O-SO₃]
[1,2,3-Trimethylimidazolium][Me-(O-CH₂-CH₂)₄-O-SO₃]
[1,2,3-Trimethylimidazolium][Me-(O-CH₂-CH₂)₅-O-SO₃]
[1,2,3-Trimethylimidazolium][Et-(O-CH₂-CH₂)₂-O-SO₃]
[1,2,3-Trimethylimidazolium][Et-(O-CH₂-CH₂)₃-O-SO₃]
[1,2,3-Trimethylimidazolium][CH₃-(CH₂)₃-O-SO₃]
[1,2,3-Trimethylimidazolium][CH₃-(CH₂)₇-O-SO₃]
[1-Ethyl-3-methylimidazolium][Me-(O-CH₂-CH₂)₂-O-SO₃]
[1-Ethyl-3-methylimidazolium][Me-(O-CH₂-CH₂)₃-O-SO₃]
[1-Ethyl-3-methylimidazolium][Me-(O-CH₂-CH₂)₄-O-SO₃]
[1-Ethyl-3-methylimidazolium][Me-(O-CH₂-CH₂)₅-O-SO₃]
[1-Ethyl-3-methylimidazolium][Et-(O-CH₂-CH₂)₂-O-SO₃]
[1-Ethyl-3-methylimidazolium][Et-(O-CH₂-CH₂)₃-O-SO₃]
[1-Ethyl-3-methylimidazolium][Me-(O-CH₂-CH₂)₂-O-SO₃]
[1-Ethyl-3-methylimidazolium][Me-(O-CH₂-CH₂)₃-O-SO₃]
[1-Ethyl-3-methylimidazolium][Me-(O-CH₂-CH₂)₄-O-SO₃]
[1-Ethyl-3-methylimidazolium][Me-(O-CH₂-CH₂)₅-O-SO₃]
[1-Ethyl-3-methylimidazolium][CH₃-(CH₂)₃-O-SO₃]
[1-Ethyl-3-methylimidazolium][CH₃-(CH₂)₇-O-SO₃]
[1-Butyl-3-methylimidazolium][Me-(O-CH₂-CH₂)₂-O-SO₃]
[1-Butyl-3-methylimidazolium][Me-(O-CH₂-CH₂)₃-O-SO₃]
[1-Butyl-3-methylimidazolium][Me-(O-CH₂-CH₂)₄-O-SO₃]
[1-Butyl-3-methylimidazolium][Me-(O-CH₂-CH₂)₅-O-SO₃]
[1-Butyl-3-methylimidazolium][Et-(O-CH₂-CH₂)₂-O-SO₃]
[1-Butyl-3-methylimidazolium][Et-(O-CH₂-CH₂)₃-O-SO₃]
[1-Butyl-3-methylimidazolium][CH₃-(CH₂)₃-O-SO₃]
[1-Butyl-3-methylimidazolium][CH₃-(CH₂)₇-O-SO₃]
[1-Octyl-3-methylimidazolium][Me-(O-CH₂-CH₂)₂-O-SO₃]
[1-Octyl-3-methylimidazolium][Me-(O-CH₂-CH₂)₃-O-SO₃]
[1-Octyl-3-methylimidazolium][Me-(O-CH₂-CH₂)₄-O-SO₃]
[1-Octyl-3-methylimidazolium][Me-(O-CH₂-CH₂)₅-O-SO₃]
[1-Octyl-3-methylimidazolium][Et-(O-CH₂-CH₂)₂-O-SO₃]
[1-Octyl-3-methylimidazolium][Et-(O-CH₂-CH₂)₃-O-SO₃]
[1-Octyl-3-methylimidazolium][CH₃-(CH₂)₃-O-SO₃]
[1-Octyl-3-methylimidazolium][CH₃-(CH₂)₇-O-SO₃]
[1-Methylpyridinium][Me-(O-CH₂-CH₂)₂-O-SO₃]
[1-Methylpyridinium][Me-(O-CH₂-CH₂)₃-O-SO₃]
[1-Methylpyridinium][Me-(O-CH₂-CH₂)₄-O-SO₃]
[1-Methylpyridinium][Me-(O-CH₂-CH₂)₅-O-SO₃]
[1-Methylpyridinium][Et-(O-CH₂-CH₂)₂-O-SO₃]
[1-Methylpyridinium][Et-(O-CH₂-CH₂)₃-O-SO₃]
[1-Methylpyridinium][CH₃-(CH₂)₃-O-SO₃]
[1-Methylpyridinium][CH₃-(CH₂)₇-O-SO₃]
[Trioctylmethylammonium][Me-(O-CH₂-CH₂)₂-O-SO₃]
[Trioctylmethylammonium][Me-(O-CH₂-CH₂)₃-O-SO₃]
[Trioctylmethylammonium][Me-(O-CH₂-CH₂)₄-O-SO₃]
[Trioctylmethylammonium][Me-(O-CH₂-CH₂)₅-O-SO₃]
[Trioctylmethylammonium][Et-(O-CH₂-CH₂)₂-O-SO₃]
[Trioctylmethylammonium][Et-(O-CH₂-CH₂)₃-O-SO₃]
[Trioctylmethylammonium][CH₃-(CH₂)₃-O-SO₃]
[Trioctylmethylammonium][CH₃-(CH₂)₇-O-SO₃]

Die Erfindung wird durch die nachfolgenden Beispiele erläutert, ohne jedoch darauf beschränkt zu sein.

### Beispiele

### Beispiel 1: Herstellung von 1-Ethyl-3-methylimidazolium 2-(2-methoxyethoxy)ethylsulfat ([EMIM][Me(EG)₂OSO₃]

In einem 100 ml Schlenkkolben wurden unter Schutzgas 16,27 g (0,129 Mol) Dimethylsulfat unter Eiskühlung langsam zu 12,40 g (0,129 Mol) Ethylimidazol getropft. Der Ansatz wurde über Nacht bei Raumtemperatur nachgerührt. Nach der Zugabe von 30,92 g (0,258 Mol; 2 eq.) Diethylenglykolmonomethylether wurden die flüchtigen Anteile 5 Stunden bei 160°C in einer Destillationsapparatur entfernt. Nach Trocknung des Ansatzes im Hochvakuum wurde 1-Methyl-3-ethylimidazolium 2-(2-methoxyethoxy)ethylsulfat als niedrigviskose, schwachgelbe Flüssigkeit in quantitativer Ausbeute erhalten.

¹H-NMR (300 MHz, CDCl₃):
9.06 (s, 1H, NCHN), 7.37 (m, 2H, NCHCHN), 4.07 (q, ³J=7.5 Hz, 2H, NCH₂CH₃), 3.92 (m, 2H, CH₂OSO₃), 3.76 (s, 3H, NCH₃), 3.49 (m, 2H, CH₂CH₂OSO₃), 3.38 (m, 2H, CH₂CH₂OCH₃), 3.27 (m, 2H, CH₂OCH₃), 3.07 (s, 3H, OCH₃), 1.31 (t, ³J=7.5 Hz, 3H, NCH₂CH₃) ppm.
¹³C-NMR (75 MHz, CDCl₃):
136.9 (NCHN), 124.1/122.4 (NCHCHN), 72.0/70.4/70.0/66.5 (CH₂CH₂OCH₂CH₂OSO₃), 59.0 (OCH₃), 45.2 (NCH₂CH₃), 36.4 (NCH₃), 15.7 (NCH₂CH₃) ppm.

### Beispiel 2: Herstellung von 1,3-Dimethylimidazolium 2-methoxyethylsulfat ([MMIM][Me(EG)OSO₃]

In einem 100 ml Schlenkkolben wurden unter Schutzgas 32,16 g (0,255 Mol) Dimethylsulfat unter Eiskühlung langsam zu 20,94 g (0,255 Mol) Methylimidazol getropft. Der Ansatz wurde über Nacht bei Raumtemperatur nachgerührt. Nach der Zugabe von 30,82 g (0,510 Mol; 2 eq.) 2-Methoxyethanol wurde 48 Stunden bei 130°C unter Rückfluss erhitzt. Die flüchtigen Anteile wurden im Hochvakuum bei 80°C entfernt. Das Produkt, 1,3-Dimethylimidazolium 2-methoxyethylsulfat, wurde als niedrigviskose, schwachgelbe Flüssigkeit in quantitativer Ausbeute erhalten.

¹H-NMR (300 MHz, CD₃CN) :
8.89 (s, 1H, NCHN), 7.47 (d, ³J=1.5 Hz, 2H, NCHCHN), 3.94 (m, 2H, CH₂OSO₃), 3.87 (s, 6H, NCH₃), 3.53 (m, 2H, CH₂CH₂OSO₃), 3.27 (s, 3H, CH₃O) ppm.
¹³C-NMR (75 MHz, CD₃CN) :
137.0 (NCHN), 117.2 (NCHCHN), 70.6 (CH₂CH₂OSO₃), 65.2 (CH₂CH₂OSO₃), 57.4 (CH₃O), 35.3 (NCH₃) ppm.

### Beispiel 3: Herstellung von 1-Ethyl-3-methylimidazolium octylsulfat ([EMIM][OcOSO₃]

In einem 250 ml Schlenkkolben wurden unter Schutzgas 58,75 g (0,381 Mol) Diethylsulfat unter Eiskühlung langsam zu 31,28 g (0,381 Mol) Methylimidazol getropft. Der Ansatz wurde über Nacht bei Raumtemperatur nachgerührt. Nach der Zugabe von 62,72 g (0,482 Mol; 1,26 eq.) 1-Octanol und 0,67 g mit Aceton gewaschenem und im Hochvakuum getrocknetem Lewatit K-2629 (saurer Ionenaustauscher) wurde über Nacht bei 140°C umgesetzt und gleichzeitig unter Durchleitung eines Argonstroms das gebildete Koppelprodukt Ethanol aus der Reaktionsmischung entfernt. Die verbleibenden flüchtigen Anteile wurden im Anschluss im Hochvakuum bei 80°C entfernt. Das Produkt, 1-Ethyl-3-methylimidazolium 1-octylsulfat, wurde als mittelviskose, schwachgelbe Flüssigkeit in quantitativer Ausbeute erhalten.

¹H-NMR (300 MHz, d₆-DMSO) :
9.14 (s, 1H, NCHN), 7.80/7.71 (2×s, 2×1H, NCHCHN), 4.20 (q, 2H, NCH₂CH₃), 3.86 (s, 3H, NCH₃), 3.69 (t, ³J=6.6 Hz, 2H, CH₂OSO₃), 1.47-1.39 (k.B., 5H, NCH₂CH₃/ CH₂CH₂OSO₃ ), 1.24 (br. s, 10H, CH₃(CH₂)₅CH₂CH₂OSO₃), 0.86 (t, ³J=6.8 Hz, CH₃(CH₂)₇OSO₃) ppm.

### Beispiel 4: Herstellung von 1-Butyl-3-methylimidazolium octylsulfat ([BMIM][OcOSO₃]

In einem 250 ml Schlenkkolben wurden unter Schutzgas 48,04 g (0,381 Mol) Dimethylsulfat unter Eiskühlung langsam zu 47,31 g (0,381 Mol) Butylimidazol getropft. Der Ansatz wurde über Nacht bei Raumtemperatur nachgerührt. Nach der Zugabe von 62,72 g (0,482 Mol; 1,26 eq.) 1-Octanol und 0,67 g mit Aceton gewaschenem und im Hochvakuum getrocknetem Dowex Ionenaustauscher wurde über Nacht bei 140°C umgesetzt und gleichzeitig unter Durchleitung eines Argonstroms das gebildete Koppelprodukt Methanol aus der Reaktionsmischung entfernt. Die verbleibenden flüchtigen Anteile wurden im Anschluss im Hochvakuum bei 80°C entfernt. Das Produkt, 1-Butyl-3-methylimidazolium 1-octylsulfat, wurde als viskose, schwachgelbe Flüssigkeit in quantitativer Ausbeute erhalten. Das Produkt enthält keine nachweisbaren Mengen an Cl-Verunreinigung (AgNO₃-Test; <3 ppm).

¹H-NMR (300 MHz, d6-DMSO): δ = 9.16 (s, 1H, N-C**H**-N), 7.80, 7.72 (zwei s, zweimal 1H, N-C**H**), 4.18 (t, 3J=7.1 Hz, 2H, N-C**H**₂-), 3.86 (s, 3H, N-C**H**₃), 3.71 (t, 3J=6.6 Hz, 2H, S-O-C**H**₂), 3.71 (p, 3J=7.3 Hz, 2H, N-CH₂-C**H**₂-), 1.47 (mult.., 2H, N-CH₂-CH₂-C**H**₂-), 1.22 (mult., 12H, S-O-CH₂-(C**H**₂)₆-), 0.81-0.90 (zwei tr, zweimal 3H, -C**H**₃) ppm.
¹³C-NMR (75 MHz, d6-DMSO): δ = 136.9, 123.9, 122.6, 66.0, 55.2, 48.8 , 36.0, 31.8, 31.6, 29.4, 29.1, 25.9, 22.4, 19.1, 14.2,13.5 ppm.

### Vergleichsversuch zur Herstellung von ([BMIM][OcOSO₃] nach dem Stand der Technik [Green Chem. 2002, 4(4), Seiten 400-404]

84,55 g (0,484 mol) 1-Butyl-3-methylimidazolium chlorid [BMIM]Cl und 101,1 g Na[*n*-C₈H₁₇O-SO₃] werden in 200ml heißem Wasser gelöst. Das Wasser wird langsam im Vakuum entfernt und ein weißer Niederschlag wird isoliert. Dieser wird mit 200 ml absolut wasserfreiem CH₂Cl₂ extrahiert und der zurückbleibende Niederschlag wird abfiltriert und noch zweimal mit je 100 ml absolutem Methylenchlorid gewaschen. Die vereinigten Methylenchlorid-Phasen werden dreimal mit 30 ml-Portionen Wasser gewaschen, bis der Chloridgehalt des Waschwassers unterhalb der Nachweisgrenze liegt (AgNO₃-Test). Die Methylenchlorid-Lösung wird über Na₂SO₄ getrocknet und im Vakuum eingeengt. 111,0 g (0,319 mmol, 66% Ausbeute) des chloridfreien Produkts werden in Form einer gelblichen Flüssigkeit erhalten.

Analytische Daten wie in Beispiel 4.

## Patentansprüche

1. Verfahren zur Herstellung ionischer Flüssigkeiten der allgemeinen Formel [Kation][R'-O-SO₃], in der R' eine Gruppe der allgemeinen Formel R⁴-[X(-CH₂-)ₙ]ₘ darstellt, in der n eine Zahl zwischen 0 und 12 repräsentiert, m unabhängig von n eine Zahl zwischen 1 und 400 darstellt, X ausgewählt ist aus der Gruppe bestehend aus Sauerstoff, Schwefel, Selen, einer Einfachbindung oder einer Gruppe der allgemeinen Formel -O-Si(CH₃)₂-O-, -O-Si(CH₂CH₃)₂-O-, -O-Si(OCH₃)₂-O-, -O-Si(O-CH₂CH₃)₂-O-, -P(Phenyl)-, -PR''- und R⁴ eine lineare oder verzweigte, gesättigte oder ungesättigte, aliphatische oder alicyclische, unfunktionalisierte oder mit einer oder mehreren Gruppen Y funktionalisierte Alkylgruppe mit 1 bis 36 Kohlenstoffatomen darstellt wobei Y eine -OH, -OR'', -COOH, -COOR'', -NH₂, -SO₄, -F, -Cl, -Br, -I oder -CN -Gruppe ist und R'' eine verzweigte oder lineare Kohlenwasserstoffkette mit 1 bis 12 Kohlenstoffatomen repräsentiert, **dadurch gekennzeichnet, dass**
ein Salz der allgemeinen Formel [Kation][CH₃-O-SO₃] oder [Kation][CH₃₋CH₂-O-SO₃] mit wenigstes einem Alkohol der allgemeinen Formel R'-OH umgesetzt wird, wobei
das [Kation] ausgewählt ist aus der Gruppe bestehend aus
- quarternären Ammonium-Kationen der allgemeinen Formel
[NR¹R²R³R]⁺,
- Phosphonium-Kationen der allgemeinen Formel
[PR¹R²R³R]⁺,
- Imidazolium-Kationen der allgemeinen Formel 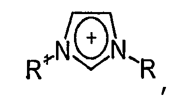 wobei der Imidazol-Kern substituiert sein kann mit wenigstens einer Gruppe, die ausgewählt ist aus C₁-C₆-Alkyl-, C₁-C₆-Alkoxy-, C₁-C₆₋Aminoalkyl-, C₅-C₁₂-Aryl- oder C₅-C₁₂-Aryl-C₁-C₆-Alkylgruppen,
- Pyridinium-Kationen der allgemeinen Formel 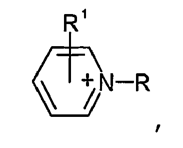 wobei der Pyridin-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆ -Alkylgruppen,
- Pyrazolium-Kationenen der allgemeinen Formel 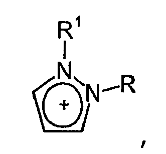 wobei der Pyrazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆-Alkylgruppen,
- und Triazolium-Kationenen der allgemeinen Formel 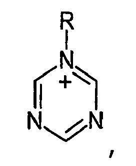 wobei der Triazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅₋C₁₂-Aryl- oder C₅-C₁₂-Aryl-C₁-C₆-Alkylgruppen,
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
und der Rest R ausgewählt ist aus
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenenatomen substituiert sein können, und R' die oben angegebene Bedeutung hat.

2. Verfahren gemäß Anspruch 1 **dadurch gekennzeichnet, dass** das Salz der Formel [Kation][CH₃-O-SO₃] durch Alkylierung eines Pyridins, Imidazols, Phosphans, Amins , Pyrazols oder Triazols mit Dimethylsulfat erhältlich ist.

3. Verfahren gemäß Anspruch 1 **dadurch gekennzeichnet, dass** das Salz der Formel [Kation][CH₃-CH₂-O-SO₃] durch Alkylierung eines Pyridins, Imidazols, Phosphans, Amins , Pyrazols oder Triazols mit Diethylsulfat erhältlich ist.

4. Verfahren gemäß Ansprüchen 1 bis 3, **dadurch gekennzeichnet, dass** die Umsetzung des Salzes der Formel [Kation][CH₃-O-SO₃] und des Salzes der Formel [Kation][CH₃-CH₂-O-SO₃] mit 1 bis 10 Äquivalenten, bevorzugt mit 1 bis 3 Äquivalenten, besonders bevorzugt mit 1 bis 2 Äquivalenten des Alkohols R'-OH erfolgt.

5. Verfahren gemäß Ansprüchen 1 bis 4, **dadurch gekennzeichnet, dass** die Umsetzung des Salzes der Formel [Kation][CH₃-O-SO₃] und des Salzes der Formel [Kation][CH₃-CH₂-O-SO₃] mit dem Alkohol R'-OH bei einer Temperatur von 0 bis 250 °C, bevorzugt 60 bis 180 °C, besonders bevorzugt 80 bis 160 °C erfolgt.

6. Verfahren gemäß Ansprüchen 1 bis 5, **dadurch gekennzeichnet, dass** de Umsetzung des Salzes der Formel [Kation][CH₃-O-SO₃] und des Salzes der Formel [Kation][CH₃-CH₂-O-SO₃] mit dem Alkohol R'-OH in Anwesenheit eines Katalysators erfolgt.

7. Verfahren gemäß Anspruch 6, **dadurch gekennzeichnet, dass** der Katalysator eine feste oder flüssige Base oder eine feste oder flüssige Säure ist.

8. Verfahren gemäß einem oder mehreren der Ansprüche 1 bis 7, **dadurch gekennzeichnet, dass** das gebildete Koppelprodukt Methanol und Ethanol im Anschluss an die Umsetzung mittels Destillation entfernt wird.

9. Verfahren gemäß einem oder mehreren der Ansprüche 1 bis 7, **dadurch gekennzeichnet, dass** das gebildete Koppelprodukt Methanol und Ethanol während der Umsetzung mittels Destillation aus dem Reaktionsgemisch entfernt wird.

10. Verfahren nach irgendeinem der Ansprüche 1 bis 9, **dadurch gekennzeichnet, dass** zwei, drei oder vier verschiedene Alkohole der Formel R'-OH mit dem Salz der allgemeinen Formel [Kation][CH₃-O-SO₃] oder [Kation][CH₃-CH₂-O-SO₃] umgesetzt werden.

## Claims

1. A process for the preparation of ionic liquids of general formula [cation][R'-O-SO₃] in which R' is a group of general formula R⁴-[X(-CH₂-)ₙ]ₘ wherein n represents a number of from 0 to 12, m independently of n represents a number of from 1 to 400, X is selected from the group consisting of oxygen, sulfur, selenium, a single bond or a group of general formula -O-Si(CH₃)₂-O-, -O-Si(CH₂CH₃)₂-O-, -O-Si(OCH₃)₂-O-, -O-Si(O-CH₂CH₃)₂-O-, -P(phenyl)-, -PR"-, and R⁴ represents a linear or branched, saturated or unsaturated, aliphatic or alicyclic alkyl group which is non-functionalized or functionalized with one or more groups Y and which includes from 1 to 36 carbon atoms, wherein Y is an -OH, -OR", -COOH, -COOR", -NH₂, -SO₄, -F, -Cl, -Br, -I or -CN group, and R" is a branched or linear hydrocarbon chain with 1 to 12 carbon atoms, **characterized in that**
a salt of general formula [cation][CH₃-O-SO₃] or [cation][CH₃-CH₂-O-SO₃] is reacted with at least one alcohol of general formula R'-OH, wherein
said [cation] is selected from the group consisting of
- quaternary ammonium cations of general formula
[NR¹R²R³R]⁺,
- phosphonium cations of general formula
[PR¹R²R³R]⁺,
- imidazolium cations of general formula 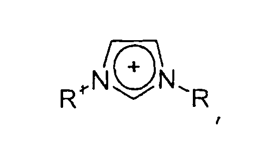
wherein the imidazole nucleus may be substituted with at least one group selected from C₁-C₆ alkyl, C₁-C₆ alkoxy, C₁-C₆ aminoalkyl, C₅-C₁₂ aryl or C₅₋C₁₂-aryl-C₁-C₆-alkyl groups;
- pyridinium cations of general formula 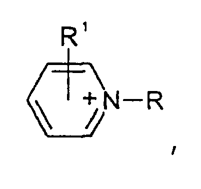
wherein the pyridine nucleus may be substituted with at least one group selected from C₁-C₆ alkyl, C₁-C₆ alkoxy, C₁-C₆ aminoalkyl, C₅-C₁₂ aryl or C₅₋C₁₂-aryl-C₁-C₆-alkyl groups;
- pyrazolium cations of general formula 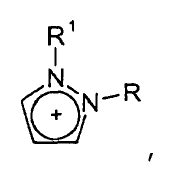
wherein the pyrazole nucleus may be substituted with at least one group selected from C₁-C₆ alkyl, C₁-C₆ alkoxy, C₁-C₆ aminoalkyl, C₅-C₁₂ aryl or C₅₋C₁₂-aryl-C₁-C₆-alkyl groups; and
- triazolium cations of general formula 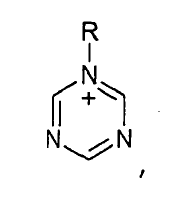 wherein the triazole nucleus may be substituted with at least one group selected from C₁-C₆ alkyl, C₁-C₆ alkoxy, C₁-C₆ aminoalkyl, C₅-C₁₂ aryl or C₅₋C₁₂-aryl-C₁-C₆-alkyl groups; and
the residues R¹, R², R³ are independently selected from the group consisting of
- hydrogen;
- linear or branched, saturated or unsaturated, aliphatic or alicyclic alkyl groups having from 1 to 20 carbon atoms;
- heteroaryl, heteroaryl-C₁-C₆-alkyl groups having from 3 to 8 carbon atoms in the heteroaryl residue and at least one heteroatom selected from N, O and S which may be substituted with at least one group selected from C₁-C₆ alkyl groups and/or halogen atoms;
- aryl, aryl-C₁-C₆-alkyl groups having from 5 to 12 carbon atoms in the aryl residue which may optionally be substituted with at least one C₁-C₆ alkyl group and/or halogen atom;
and the residue R is selected from
- linear or branched, saturated or unsaturated, aliphatic or alicyclic alkyl groups having from 1 to 20 carbon atoms;
- heteroaryl-C₁-C₆-alkyl groups having from 3 to 8 carbon atoms in the aryl residue and at least one heteroatom selected from N, O and S which may be substituted with at least one C₁-C₆ alkyl group and/or halogen atom;
- aryl-C₁-C₆-alkyl groups having from 5 to 12 carbon atoms in the aryl residue which may optionally be substituted with at least one C₁-C₆ alkyl group and/or halogen atom;
and R' has the meaning as defined above.

2. The process according to claim 1, **characterized in that** the salt of formula [cation][CH₃-O-SO₃] is obtainable by alkylating a pyridine, imidazole, phosphane, amine, pyrazole or triazole with dimethyl sulfate.

3. The process according to claim 1, **characterized in that** the salt of formula [cation][CH₃-CH₂-O-SO₃] is obtainable by alkylating a pyridine, imidazole, phosphane, amine, pyrazole or triazole with diethyl sulfate.

4. The process according to claims 1 to 3, **characterized in that** the reaction of the salt of formula [cation][CH₃-O-SO₃] and of the salt of formula [cation][CH₃-CH₂-O-SO₃] is effected with 1 to 10 equivalents, preferably 1 to 3 equivalents, more preferably 1 to 2 equivalents, of the alcohol R'-OH.

5. The process according to claims 1 to 4, **characterized in that** the reaction of the salt of formula [cation][CH₃-O-SO₃] and of the salt of formula [cation][CH₃-CH₂-O-SO₃] with the alcohol R'-OH is effected at a temperature of from 0 to 250 °C, preferably 60 to 180 °C, more preferably 80 to 160 °C.

6. The process according to claims 1 to 5, **characterized in that** the reaction of the salt of formula [cation][CH₃-O-SO₃] and of the salt of formula [cation][CH₃-CH₂-O-SO₃] with the alcohol R'-OH is effected in the presence of a catalyst.

7. The process according to claim 6, **characterized in that** said catalyst is a solid or liquid base or a solid or liquid acid.

8. The process according to one or more of claims 1 to 7, **characterized in that** the by-product methanol and ethanol formed is removed by distillation following the reaction.

9. The process according to one or more of claims 1 to 7, **characterized in that** the by-product methanol and ethanol formed is removed by distillation during the reaction.

10. The process according to any of claims 1 to 9, **characterized in that** two, three or four different alcohols of formula R'-OH are reacted with the salt of general formula [cation][CH₃-O-SO₃] or [cation][CH₃-CH₂-O-SO₃].

## Revendications

1. Procédé de préparation de liquides ioniques de formule générale [cation] [R'-O-SO₃], dans laquelle R' représente un groupe de formule générale R⁴⁻[X(-CH₂-)ₙ]ₘ, dans laquelle n représente un nombre entre 0 et 12, m représente, indépendamment de n, un nombre entre 1 et 400, X est choisi dans le groupe formé par l'oxygène, le soufre, le sélénium, une liaison simple ou un groupe de formule générale -O-Si(CH₃)₂-O-, -O-Si(CH₂CH₃)₂-O-, -O-Si(OCH₃)₂-O-, -O-Si(O-CH₂CH₃)₂-O-, -P(phényle) -, -PR"- et R⁴ représente un groupe alkyle comportant 1 à 36 atomes de carbone, linéaire ou ramifié, saturé ou insaturé, aliphatique ou alicyclique, non fonctionnalisé ou fonctionnalisé avec un ou plusieurs groupes Y, dans lequel Y est un groupe -OH, -OR", -COOH, -COOR", -NH₂, -SO₄, -F, -Cl, -Br, -I ou -CN et R" représente une chaîne hydrocarbonée ramifiée ou linéaire comportant 1 à 12 atomes de carbone, **caractérisé en ce que** l'on fait réagir
un sel de formule générale [cation][CH₃-O-SO_{3]} ou [cation][CH₃-CH₂-O-SO₃] avec au moins un alcool de formule générale R'-OH,
le [cation] étant choisi dans le groupe formé par
- les cations de type ammonium quaternaires de formule générale
[NR¹R²R³R⁴]⁺
- les cations de type phosphonium de formule générale
[PR¹R²R³R⁴]⁺
- les cations de type imidazolium de formule générale 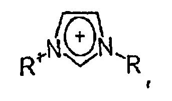 dans laquelle le noyau imidazole peut être substitué par au moins un groupe qui est choisi parmi les groupes alkyles en C₁ à C₆, alcoxy en C₁ à C₆, aminoalkyle en C₁ à C₆, aryle en C₅ à C₁₂ ou (aryle en C₅ à C₁₂)(alkyle en C₁ à C₆),
- les cations de type pyridinium de formule générale 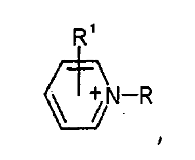 dans lesquels le noyau pyridine peut être substitué par au moins un groupe qui est choisi parmi les groupes alkyle en C₁ à C₆, alcoxy en C₁ à C₆, aminoalkyle en C₁ à C₆, aryle en C₅ à C₁₂ ou (aryle en C₅ à C₁₂)(alkyle en C₁ à C₆),
- les cations de type pyrazolium de formule générale 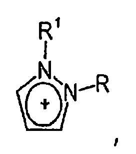 dans lesquels le noyau pyrazole peut être substitué par au moins un groupe qui est choisi parmi les groupes alkyle en C₁ à C₆, alcoxy en C₁ à C₆, aminoalkyle en C₁ à C₆, aryle en C₅ à C₁₂ ou (aryle en C₅ à C₁₂)(alkyle en C₁ à C₆),
- des cations triazolium de la formule générale 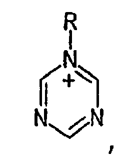 dans lesquels le noyau triazole peut être substitué par au moins un groupe qui est choisi parmi les groupes alkyle en C₁ à C₆, alcoxy en C₁ à C₆, aminoalkyle en C₁ à C₆, aryle en C₅ à C₁₂ ou (aryle en C₅ à C₁₂)(alkyle en C₁ à C₆),
et les radicaux R¹, R², R³ sont choisis indépendamment les uns des autres dans le groupe formé par
- l'hydrogène
- les groupes alkyle comportant 1 à 20 atomes de carbone, linéaires ou ramifiés, saturés ou insaturés, aliphatiques ou alicycliques ;
- des groupes hétéroaryle, hétéroaryle-(alkyle en C₁ à C₆) avec 3 à 8 atomes de carbone dans le radical hétéroaryle et au moins un hétéroatome choisi parmi N, O et S, qui peuvent être substitués par au moins un groupe choisi parmi les groupes alkyle en C₁ à C₆ et/ou des atomes d'halogène ;
- des groupes aryle, aryle-(alkyle en C₁ à C₆) avec 5 à 12 atomes de carbone dans le radical aryle, qui peuvent être substitués le cas échéant par au moins un groupe alkyle en C₁ à C₆ et/ou par un atome d'halogène ;
et le radical R est choisi parmi
- des groupes alkyle comportant 1 à 20 atomes de carbone, linéaires ou ramifiés, saturés ou insaturés, aliphatiques ou alicycliques ;
- des groupes hétéroaryle-(alkyle en C₁ à C₆) avec 3 à 8 atomes de carbone dans le radical aryle et au moins un hétéroatome choisi parmi N, O et S, qui peuvent être substitués par au moins un groupe alkyle en C₁ à C₆ et/ou des atomes d'halogènes ;
- des groupes aryle-(alkyle en C₁ à C₆) avec 5 à 12 atomes de carbone dans le radical aryle, qui peuvent être substitués le cas échéant par au moins un groupe alkyle en C₁ à C₆ et/ou un atome d'halogène ;
et R' a la signification donnée ci-dessus.

2. Procédé selon la revendication 1, **caractérisé en ce que** le sel de formule [cation][CH₃-O-SO₃] peut être obtenu par alkylation d'une pyridine, d'un imidazole, d'un phosphane, d'une amine, d'un pyrazole ou d'un triazole avec du sulfate de diméthyle.

3. Procédé selon la revendication 1, **caractérisé en ce que** le sel de formule [cation][CH₃-CH₂-O-SO₃] peut être obtenu par alkylation d'une pyridine, d'un imidazole, d'un phosphane, d'une amine, d'un pyrazole ou d'un triazole avec du sulfate de diéthyle.

4. Procédé selon les revendications 1 à 3, **caractérisé en ce que** la réaction du sel de formule [cation][CH₃-O-SO₃] et du sel de formule [cation][CH₃-CH₂-O-SO₃] s'effectue avec 1 à 10 équivalents, de préférence avec 1 à 3 équivalent, de façon particulièrement préférée avec 1 à 2 équivalents d'alcool R'-OH.

5. Procédé selon les revendications 1 à 4, **caractérisé en ce que** la réaction du sel de formule [cation][CH₃-O-SO₃] et du sel de formule [cation][CH₃-CH₂-O-SO₃] avec l'alcool R'-OH s'effectue à une température de 0 à 250 °C, de préférence de 60 à 180 °C, de façon particulièrement préférée de 80 à 160 °C.

6. Procédé selon les revendications 1 à 5, **caractérisé en ce que** la réaction du sel de la formule [cation][CH₃-O-SO₃] et du sel de formule [cation][CH₃-CH₂-O-SO₃] avec l'alcool R'-OH s'effectue en présence d'un catalyseur.

7. Procédé selon la revendication 6, **caractérisé en ce que** le catalyseur est une base solide ou liquide ou un acide solide ou liquide.

8. Procédé selon l'une ou plusieurs des revendications 1 à 7, **caractérisé en ce que** le produit de couplage obtenu, méthanol ou éthanol, est éliminé par distillation après la réaction.

9. Procédé selon l'une ou plusieurs des revendications 1 à 7, **caractérisé en ce que** le produit de couplage obtenu, méthanol ou éthanol, est éliminé du mélange réactionnel par distillation pendant la réaction.

10. Procédé selon l'une quelconque des revendications 1 à 9, **caractérisé en ce que** l'on fait réagir deux, trois ou quatre alcools différents de formule R'-OH avec le sel de formule générale [cation][CH₃-O-SO₃] ou [cation][CH₃-CH₂-O-SO₃].
